# EUROPEAN PATENT APPLICATION

(11) **EP 3 299 794 A1**
(43) Date of publication of application: **28.03.2018**
(21) Application number: 16189852.3
(22) Date of filing: 21.09.2016
(51) Int. Cl.: G01N 5/04, B01L 3/00, G01N 35/00

(54) **AUTOMATED SCHEDULER FOR LABORATORY EQUIPMENT**

(71) Applicant: F. Hoffmann-La Roche AG, 4070 Basel (CH); Roche Diagnostics GmbH, 68305 Mannheim (DE)
(72) Inventor: Janner, Gabriele Piero, 8050 Zürich (CH); Jost, Friedrich, 6300 Zug (CH); Korner, Stephan, 6330 Cham (CH); Oosterbroek, Rijk Edwin, 6330 Cham (CH)
(74) Representative: Peterreins Schley

(57) **Abstract**

In one aspect of the present disclosure, a computer-implemented method includes receiving at least one order, generating one or more optimization problem instances using one or more resource descriptions of automated laboratory equipment, one or more protocol descriptions of the automated laboratory equipment, an objective function and the at least one order, the method further comprising providing the one or more optimization problem instances as input to a constraint optimization solver and processing the one or more optimization problem instances by the constraint optimization solver to generate the schedule of operations to carry out the at least one order on the automated laboratory equipment.

## Description

### Technical Field

This disclosure relates to methods and systems for providing a schedule of operations for automated laboratory equipment.

### Background

In today's laboratory environments, automated laboratory equipment for performing various tasks can be deployed. The automated laboratory equipment can receive samples and orders. The orders describe which protocol(s) to carry out on each sample. It can happen that multiple samples are processed in parallel, e.g., to increase throughput or reduce a time-to-result. For carrying out the protocols, numerous resources can be required, such as instrument resources (e.g., transport systems for sample holders and reaction vessels, robotic arms with pipettes to transfer and dose sample and/or reagent liquids, fluid mixers, incubator positions, photodetector positions etc.) and consumable resources (e.g., reagents, disposable fluid carriers such as tubes, reaction vessels, multi-well plates or other fluid carriers for sample fluids and reagents, disposable pipette tips and many more). The sequence of using the resources to process an order is called a process. Since multiple processes, each addressing a protocol, run fully or partially in parallel and share some of the resources, a pattern of resource activities of the laboratory equipment can be fairly complex. This complexity can even be increased by demanding timing-windows, for instance for chemical reactions in analyzers, and by rules defined to avoid, e.g., carry over or requested order priorities. As a consequence, the development and operation of a schedule of operations for automated laboratory equipment can be very challenging.

### Summary

In a first general aspect, a computer-implemented method for providing a schedule of operations for automated laboratory equipment includes receiving at least one order, each order requiring the execution of one or more protocols on the automated laboratory equipment, generating one or more optimization problem instances using one or more resource descriptions of the automated laboratory equipment, one or more protocol descriptions of the automated laboratory equipment, an objective function and the at least one order, the one or more resource descriptions describing the resources present in the automated laboratory equipment and capabilities and constraints of the resources, each protocol description describing a protocol the automated laboratory equipment can carry out, each protocol description including a definition of one or more protocol steps to be carried out by the automated laboratory equipment and each protocol description including one or more constraints to be observed when carrying out the protocol steps on the laboratory equipment, the objective function defining an optimization goal for a schedule of operations for the automated laboratory equipment, the method further comprising providing the one or more optimization problem instances as input to a constraint optimization solver and processing the one or more optimization problem instances by the constraint optimization solver to generate the schedule of operations to carry out the at least one order on the automated laboratory equipment.

In a second general aspect, a laboratory network includes automated laboratory equipment and a computer system configured to carry out the steps of the first general aspect.

Particular embodiments of the subject-matter of the first and second general aspects can be implemented so as to realize one or more of the following advantages.

Firstly, the techniques of the present disclosure can allow for a fast and flexible generation of a schedule of operations for automated laboratory equipment in some examples.

For example, by using an optimization problem instance which models the automated laboratory equipment and a (potentially generic) constraint optimization solver, the schedule can be generated without or with little prior knowledge regarding a structure of the schedule to be generated (i.e., the solution of the scheduling problem).

In some prior art schedulers, pre-defined templates of operations are used (e.g., templates which define at which times a particular operation can be carried out). The protocols are defined such that they can be fitted into the pre-defined templates. During run-time (e.g., upon arrival of one or more orders), the scheduler fits the respective protocol required for the orders into the pre-defined templates. The quality of the schedules of these schedulers can depend strongly on the pre-defined templates. Moreover, it might not be straightforward to modify the scheduler, e.g., to include new protocols, to operate on a different automated laboratory equipment, or to change parameters of the automated laboratory equipment (e.g., a pipetting time or an incubation duration). This process might require involvement of an experienced user who has deep knowledge about the respective automated laboratory equipment or substantial trial-and-error work to prepare new templates.

The technique of the present invention can ameliorate or even overcome these problems in some examples. The resource and protocol descriptions can be amended and adapted to different laboratory equipment, a changed design of the automated laboratory equipment or changed protocols in a relatively straightforward manner. It might not be required to provide information regarding potential solutions in the scheduling process. In addition, the constraint optimization solver can be re-run after one of the above described changes have occurred without requiring (substantial) modifications to the solver.

For instance, a first run (or plurality of runs) of the solver might generate a schedule which is optimized to yield a high throughput. At a later point in time, a high priority order might arrive in the laboratory. In this situation, a re-run of the constraint optimization solver can be triggered to adapt the schedule to the new situation. An updated schedule can process the high priority sample as quickly as possible.

As in this example, the technique of the present disclosure can allow for adaptation to a multitude of other situations in some examples. The underlying structure of the optimization problem instance generation process might remain (substantially) unchanged. Furthermore, a designer can change or complement the resource descriptions, the protocol descriptions and the objective function without considering a structure of the solutions of the optimization process.

In the same manner, a plurality of devices or a laboratory environment containing multiple automated devices can be considered. This might not be the case when using pre-defined templates for the scheduling process as in some prior art schedulers. Further examples of how the flexibility of the techniques of the present disclosure allows for an improved operation of laboratory equipment will be discussed below.

Secondly, the techniques of the present disclosure can be used to generate schedules which make automated laboratory equipment run faster, achieve a higher throughput, or run more resource efficiently (or a combination of two or more of these) in some examples.

Thirdly, the techniques of the present disclosure can be employed to simulate scheduling operations of automated laboratory equipment. In this manner, the technique of the present disclosure can be helpful in the development automated laboratory equipment. For example, a placement and layout of components of an automated device can be selected or changed based on the findings of a simulation with the techniques of the present disclosure. In the same manner, protocols, workflows or templates of operations of automated laboratory equipment can be developed by using the techniques of the present disclosure. Moreover, the design of automated laboratory equipment and protocols and workflows can be tested by using the simulations according to the present disclosure.

A number of terms are used in the present disclosure in a particular way:
The term 'automated laboratory equipment' as used herein can refer to any kind of automated or semi-automated technical device for use in laboratory work, e.g., in the clinical, chemical, biological, immunology or pharmaceutical area or the like. Such a laboratory device may comprise components to perform fluid transfer and dosing, fluid homogenization (mixing), temperature control, and measurements of chemical or physical parameters. For example, the devices can include fluid dispensing components (e.g., a pipettor or a valve), a stirrer, a tempering device, a shaker, or an agitator.

In other examples, automated laboratory equipment can include an analysis system or a work-cell of an analysis system or analyzer. For example, automated laboratory equipment can be an analyzer for analyzing a mechanical, optical, chemical or biological property of a sample. 'Automated laboratory equipment' not necessarily is located in a dedicated laboratory.

Rather, the term also includes stand-alone laboratory equipment for carrying out analytic procedures, e.g., in the clinical, chemical, biological, immunology or pharmaceutical area. For example, a benchtop device in point-of-care settings such as physician clinics or pharmacies or a device for home-use can also be automated laboratory equipment according to the present disclosure.

'Automated laboratory equipment' as used herein comprises a control unit or controller operatively which can be coupled to one or more analytical, pre- and post-analytical work cells, the control unit being operable to control the work cells. In addition, the control unit may be operable to evaluate and/or process gathered analysis data, to control the loading, storing and/or unloading of samples to and/or from any one of the analyzers, to initialize an analysis or hardware or software operations of the analysis system used for preparing the samples, sample tubes or reagents for said analysis and the like.

In one example, an automated laboratory equipment can be a transportation system configured to transport sample in a laboratory environment.

The term 'analyzer' / 'analytical work cell' as used herein encompasses any apparatus or apparatus component that can measure physical or chemical characteristics of a sample. In some examples, the device can be configured to induce a reaction of a biological sample with a reagent for obtaining a measurement value.

An analyzer can be operable to determine via various chemical, biological, physical, optical or other technical procedures a parameter value of the sample or a component thereof. An analyzer may be operable to measure said parameter of the sample or of at least one analyte and return the obtained measurement value. The list of possible analysis results returned by the analyzer comprises, without limitation, concentrations of the analyte in the sample, a digital (yes or no) result indicating the existence of the analyte in the sample (corresponding to a concentration above the detection level), optical parameters, images, cell or particle counts, DNA or RNA sequences, data obtained from mass spectroscopy of proteins or metabolites and physical, mechanical, optical, electrical or chemical parameters of various types. An analytical work cell may comprise units assisting with the pipetting, dosing, and mixing of samples and/or reagents. The analyzer may comprise a reagent holding unit for holding reagents to perform the assays. Reagents may be arranged for example in the form of containers or cassettes containing individual reagents or group of reagents, placed in appropriate receptacles or positions within a storage compartment or conveyor. It may comprise a consumable feeding unit. The analyzer may comprise a process and detection system whose workflow is optimized for certain types of analysis. Examples of such analyzer are clinical chemistry analyzers, coagulation chemistry analyzers, immunochemistry analyzers, urine analyzers, nucleic acid analyzers, used to detect the result of chemical or biological reactions or to monitor the progress of chemical or biological reactions.

The term 'workflow' as used herein encompasses any task that comprises a number of steps, such as for maintenance or operation of the system or one of its system components.

The term 'step of a workflow' or 'task of a workflow' as used herein encompasses any activity belonging to a workflow. The activity can be of an elementary or complex nature and is typically performed at or by means of a single automated laboratory equipment. A 'workflow' might include all processing steps taking place in the automated laboratory equipment, or only a subset of the tasks (e.g., those that are relevant for scheduling in some situations).

The term 'communication network' as used herein encompasses any type of wireless network, such as a WIFI, GSM, UMTS or other wireless digital network or a cable based network, such as Ethernet or the like. In particular, the communication network can implement the Internet protocol (IP). For example, the communication network comprises a combination of cable-based and wireless networks.

A 'control unit' or 'controller' controls the automated or semi-automated system in a way that the necessary steps for the processing protocols are conducted by the automated system. That means the control unit may, for example, instruct the automated system to conduct certain pipetting steps to mix the liquid biological sample with reagents, or the control unit controls the automated system to incubate the sample mixtures for a certain time etc. The control unit may receive information from a data management unit regarding which steps need to be performed with a certain sample. In some embodiments, the control unit might be integral with the data management unit or may be embodied by a common hardware. The control unit may, for instance, be embodied as a programmable logic controller running a computer-readable program provided with instructions to perform operations in accordance with a process operation plan. The control unit may be set up to control, for example, any one or more of the following operations: loading and/or wasting and/or washing of cuvettes and/or pipette tips, moving and/or opening of sample tubes and reagent cassettes, pipetting of samples and/or reagents, mixing of samples and/or reagents, washing pipetting needles or tips, washing mixing paddles, controlling of a light source, e.g. selection of the wavelength, or the like. In particular, the control unit may include a scheduler, for executing a sequence of steps within a predefined cycle time. The control unit may further determine the order of samples to be processed according to the assay type, urgency, and the like.

The term 'sample' refers to material(s) that may potentially contain an analyte of interest. The sample can be derived from a biological source, such as a physiological fluid, including blood, saliva, ocular lens fluid, cerebrospinal fluid, sweat, urine, stool, semen, milk, ascites fluid, mucous, synovial fluid, peritoneal fluid, amniotic fluid, tissue, cells, or the like. The biological sample can be pretreated prior to use, such as preparing plasma or serum from blood. Methods of treatment can involve centrifugation, filtration, distillation, dilution, concentration and/or separation of sample components including analytes of interest, inactivation of interfering components, and the addition of reagents. A sample may be used directly as obtained from the source or used following a pretreatment to modify the character of the sample. In some embodiments, an initially solid or semi-solid biological material can be rendered liquid by dissolving or suspending it with a suitable liquid medium. In some examples, the sample can be suspected to contain a certain antigen or nucleic acid.

The term 'order' includes any request for automated laboratory equipment to automatically or semi-automatically carry out a particular task. For example, an order can be a request that one or more assays are to be performed on one or more biological samples.

### Description of the Drawings

**FIG. 1** illustrates a schematic diagram of the structure of a scheduler according to the present disclosure.
**FIG. 2** schematically illustrates a schedule of operations of automated laboratory equipment according to the present disclosure.
**FIG. 3** schematically illustrates a dynamic adaptation of a schedule of operations of automated laboratory equipment according to the present disclosure.
**FIG. 4** illustrates an example modelling of a protocol according to the present disclosure.
**FIG. 5** illustrates a part of the generation process of an optimization problem instance according to the present disclosure.
**FIG. 6A** to **FIG. 6C** illustrate example schedules of operations obtained when changing the objective function according to the present disclosure.
**FIG. 7** is a schematic illustration of an example automated laboratory device according to the present disclosure.

### Detailed Description

The methods and systems for providing a schedule of operations for automated laboratory equipment will subsequently be discussed in more detail.

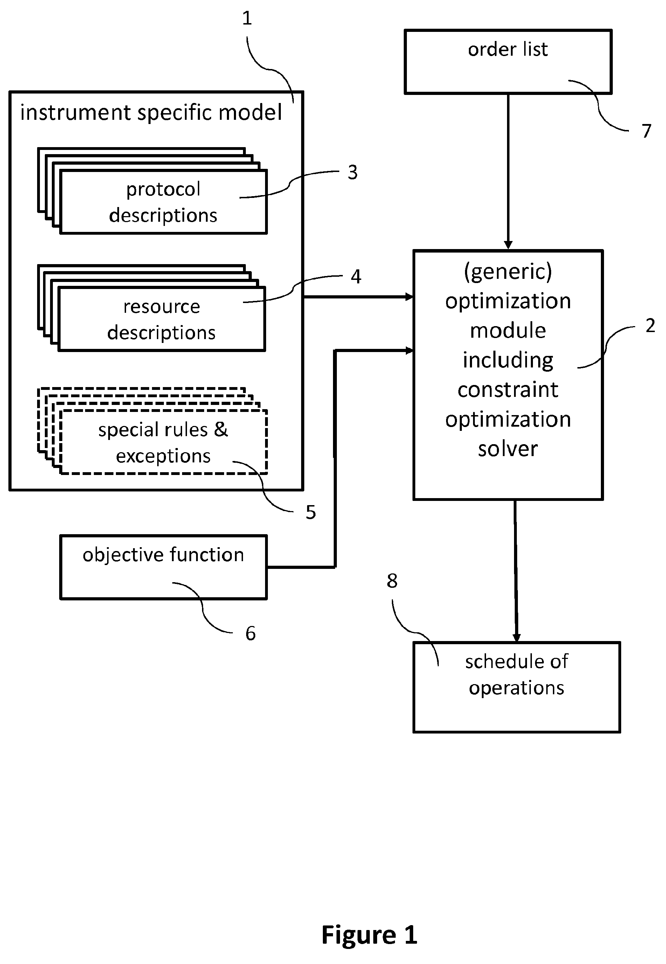

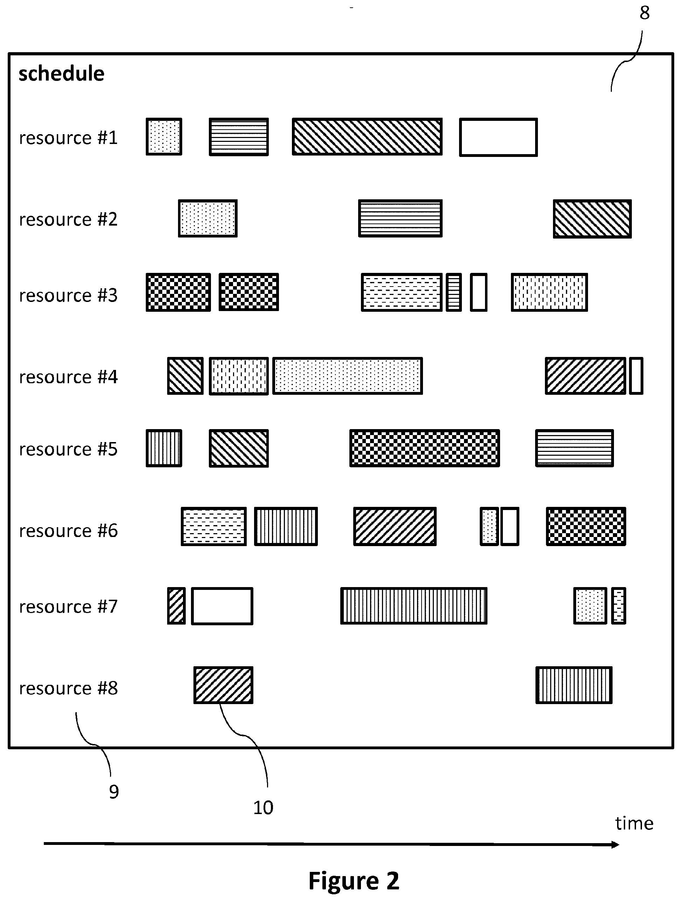

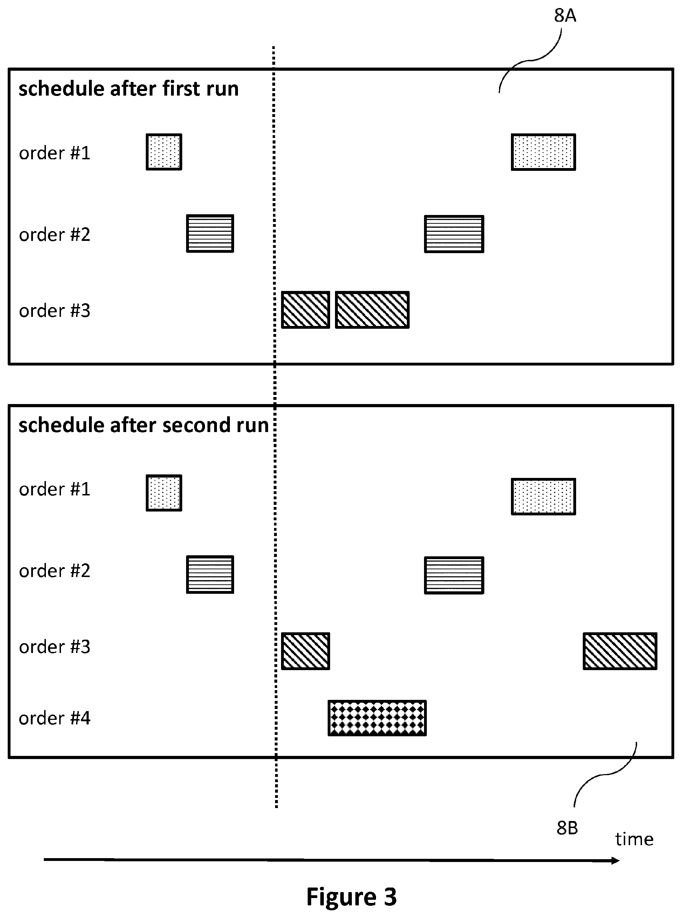

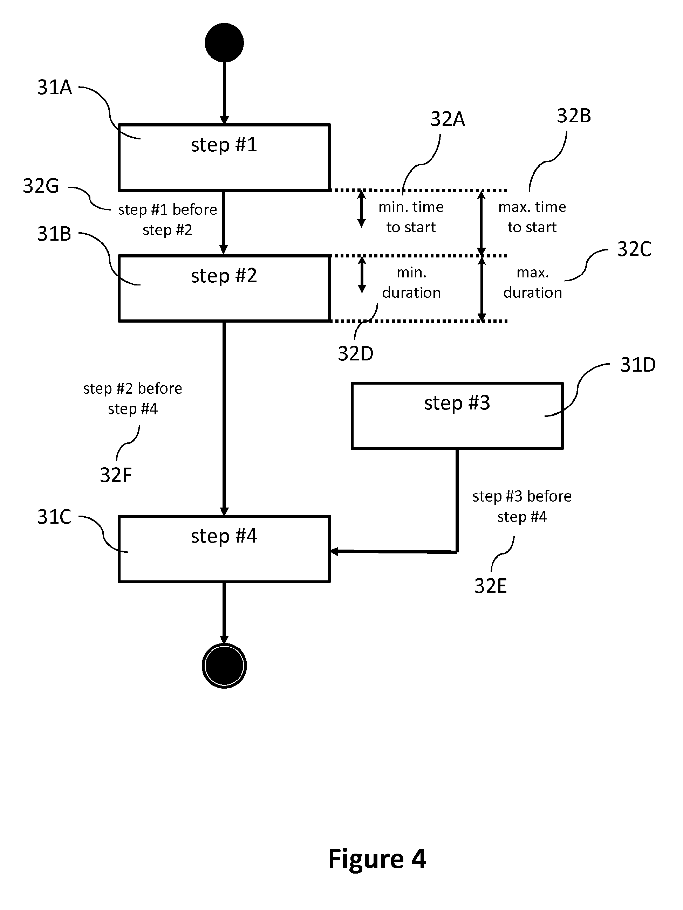

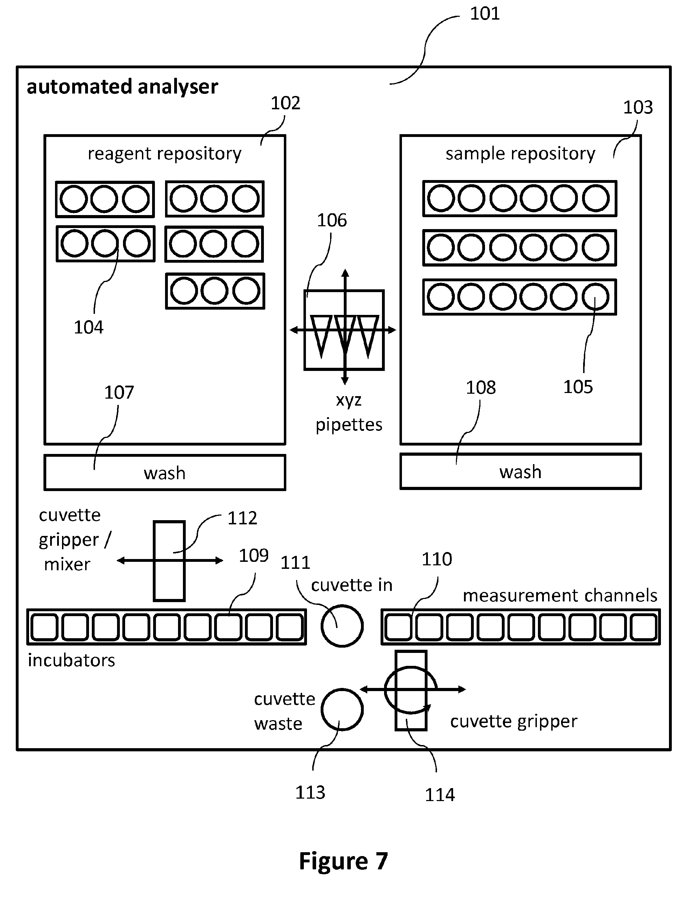

First, an overview over the structure of the scheduler will be given in connection with **FIG. 1****.** Subsequently, different aspects of the use of the techniques of the present disclosure will be discussed in connection with **FIG. 2** and **FIG. 3****.** Next, in connection with **FIG. 4** to **FIG. 6****,** additional details of the structure of the scheduler of the present disclosure will be presented. Last, an example automated laboratory device and its modelling will be discussed in more detail in connection with **FIG. 7****.**

**FIG. 1** illustrates a schematic diagram of the structure of a scheduler according to the present disclosure including a model of automated laboratory equipment 1. The model includes one or more resource descriptions 4 and one or more protocol descriptions 3. The scheduler further includes an objective function 6 and a constraint optimization solver 2 (also referred to as constraint optimization solver module herein). The constraint optimization solver 2 or constraint optimization solver module can be part of an optimization module, as shown in **FIG. 1****.** Moreover, the scheduler further receives an order list 7 including at least one order as part of the schedule generation process.

The mathematical model 1 including the resource descriptions 4 and the protocol descriptions 3, as well as the order list 7 and the objective function 6 are used to generate one or more optimization problem instances. The one or more optimization problem instances are provided to the constraint optimization solver 2 which generates a schedule of operations 8 for the one or more optimization problem instances.

The different components of the scheduler have the following functions: The order list 7 includes at least one order (e.g., a plurality of orders) to be carried out by the automated laboratory equipment, each order involving the execution of one or more protocols. For example, an order list can define a plurality of assays to be carried out by an automated analyzer on a sample or a batch of samples.

Each protocol description 3 describes a protocol the automated laboratory equipment can carry out. The protocol description 3 includes a definition of one or more protocol steps to be carried out by the automated laboratory equipment and one or more constraints to be observed when carrying out the protocol steps on the automated laboratory equipment.

For example, a protocol might be a particular assay involving multiple steps of adding reagents to a sample, homogenizing the mixture, transporting the mixture, incubating the mixture and performing optical measurements on the mixture.

The constraints can, e.g., define orders of the steps and minimum and maximum durations of the steps and of the times between two steps. The protocol descriptions 3 can describe the protocol the automated laboratory equipment can carry out independently of how the orders are scheduled in the schedule. In other words, the definition of the protocol descriptions 3 does not require information regarding the (potential) solution of a schedule generated by the solver.

Each resource description 4 describes one or more resources present in the automated laboratory equipment and capabilities and constraints of the one or more resources. For instance, a resource description 4 can describe a dispensing device (e.g., a pipettor). A constraint of this dispensing device might be that it is only capable of addressing a certain subset of fluids, e.g., only sample (certain) reagents, and /or a subset of volume ranges (e.g., only 50 to 100µl or only 110 to 200µl).

In some examples, the resource descriptions 4 are specific to a particular automated laboratory equipment. The resource descriptions 4 can be independent of how the orders are scheduled in the schedule. In other words, the definition of the resource descriptions 4 does not require information regarding the (potential) solution of a schedule generated by the solver.

The objective function 6 defines an optimization goal to be fulfilled by a schedule of operations for automated laboratory equipment.

In some examples, the techniques of the present disclosure also include obtaining one or more special rules and exceptions 5 (also short-handed as 'exceptions' herein) which define how the automated laboratory equipment should handle one or more particular situations. The special rules and exceptions 5 can also be defined as part of the resource descriptions 4 in some examples. For instance, the one or more particular situations include a situation in which a substance associated (e.g., the sample or a reagent) with a first order is carried over to a process of another order. In one example, a particular process might be extremely sensitive to contamination with a particular reagent.

In other examples, the one or more particular situations include a situation in which a resource is in a certain state after having carried out a particular task that could affect the processing of other subsequent tasks. For example, a particular sensor might not be in a state to carry out a second type of measurements directly after having carried out a first type of measurement. Further details regarding the components shown in **FIG. 1** and their implementation will be discussed below.

After having received the one or more optimization problem instances generated using the elements discussed above, the constraint optimization solver 2 processes the one or more optimization problem instances to generate the schedule of operations 8 to carry out the plurality of orders on the automated laboratory equipment. As depicted in **FIG. 1****,** the constraint optimization solver 2 can be a generic constraint optimization solver module.

For example, the constraint optimization solver module can use one or a combination of packages, containing optimization algorithms and heuristic methods. Examples of solver packages are the Cplex CP Optimizer (by IBM), the Cplex Optimizer (by IBM), the Gurobi Optimizer (by Gurobi), ECLiPSe and CBC. Examples of optimization algorithms and heuristic methods are branch and bound, branch and price and exhaustive search algorithms, local search, constructive algorithms, evolutionary algorithms, ant colony optimization and machine learning-based algorithms.

In other words, the constraint optimization solver module 2 can include no information regarding the particular automated laboratory equipment modeled. In addition, the constraint optimization solver module 2 might not receive any templates relating to solutions or part of solutions for the schedule of operations.

In other examples, the model 1 includes one or more constraints to force the constraint optimization solver to search for solutions with repetitive patterns. In this case, the exact times at which resource activities take place are not prescribed, this can be optimized by the solver, but what is defined is that the schedules shall fit a repetitive pattern. As a result, a pattern can be computed during design-time. This pattern can be fix uploaded without the need for run-time resource constraint solving. At run-time, the ordered protocols can be fit on the repetitive pattern (e.g., by the automated analyzer). Fitting the ordered protocols on the repetitive pattern can be a (simplified) optimization problem. In some examples, a generic optimizer module can be used. In other examples, dedicated software routines can be written that fit the orders onto the repetitive pattern.

The schedule of operations 8 can be further processed in multiple ways. In one example, the automated laboratory equipment can receive the schedule of operations 8 and carry out the operations according to the schedule. For instance, this can happen automatically after the schedule has been generated, or upon user input (e.g., after an operator has released the schedule). In some examples, the automated laboratory equipment can carry out the operations exactly according to the schedule of operations 8. However, in other situations the schedule of operations 8 is further processed by the automated laboratory equipment. For example, the automated laboratory equipment might not necessarily exactly follow the schedule of operations 8 but change the schedule of operations 8 (by purpose or accidentally). For instance, the automated laboratory equipment can be configured to follow the defined schedule only roughly time-wise, leaving some space for deviations.

In addition or alternatively, the schedule of operations 8 can be processed into a diagrammatic representation which can be output on a user interface for inspection by a user. For example, the schedule of operations 8 can be represented as a Gantt chart as shown in **FIG. 2** or in **FIG. 3**.

After having shortly introduced the components of the scheduler of the present disclosure, different techniques of using the techniques of the present disclosure will be discussed in connection with **FIG. 2** and **FIG. 3****.**

In general, the techniques of the present disclosure can be used to simulate a behavior of automated laboratory equipment as well as generating a schedule for actual automated laboratory equipment. The latter case includes that the generation of the schedule is performed during design-time or configuration-time of the automated laboratory equipment (e.g., before a processing of samples has started) or during run-time of the automated laboratory equipment.

In one example, the schedule of operations is generated during development- or design-time of instrument software of the automated laboratory equipment. In other examples, the schedule of operations is generated during development- or design-time of schedules of operations of the automated laboratory equipment. For example, a schedule generated during development- or design-time can be applied on automated laboratory equipment as a pre-defined schedule to be used during run-time of the automated laboratory equipment.

As can be seen in **FIG. 2****,** the schedule 8 determined by the constraint optimization solver can allocate the resources 9 of automated laboratory equipment to particular tasks at particular times. In **FIG. 2****,** the example automated laboratory equipment includes eight different resources. The steps belonging to different orders are illustrated by bars with different hatchings. For instance, resource #1 is scheduled to carry out tasks belonging to four different orders. In one example, resource #8 can be an incubator. At a predetermined point in time, the incubator is scheduled to perform an incubation operation 10 on a sample to be processed as part of a particular order. As can be seen in **FIG. 2****,** the schedule defines and order and timing of the operations of the resources 9 of the automated laboratory equipment.

As discussed above, the methods of the present disclosure can include displaying the schedule 8 to a user (e.g., on a graphical user interface). In order to do so, the techniques of the present disclosure can include transforming the information in the schedule into a graphic representation (e.g., a Gantt chart as shown in **FIG. 2** or **FIG. 3** or a 3D animation). However, the schedule can be represented in different other forms defining a sequence of the operations. In some examples the schedule might be only transmitted to a controller of the automated laboratory equipment or a simulated version of it to allow for controlling the operation according to the schedule. In these examples, no graphic representation of the information of the schedule might be generated. The schedule can merely include the information regarding the sequence of the operations to carry out the plurality of orders on the automated laboratory equipment in a machine-readable format. For example, the schedule can be a table or list defining a series of operations of the resources of automated laboratory equipment.

In some examples, the techniques of the present disclosure can involve leaving gaps in the schedule for particular resources. This can facilitate filling in new order after arrival in an existing schedule.

After having discussed the structure of the schedule, several examples of the application of the techniques of the present disclosure will be presented subsequently.

As can be seen in **FIG. 3****,** the techniques of the present disclosure can include running the constraint optimization solver multiple times to update the schedule. For example, after a plurality of orders have been received (e.g., a predefined number of orders - this technique is also called batch processing herein) or a certain time interval has elasped, the constraint optimization solver is run a first time and generates a schedule of operations 8A for automated laboratory equipment.

The automated laboratory equipment can start to process the orders according to the schedule. Then, at a later point in time, one or multiple additional orders are received. In response to receiving the additional orders, the constraint optimization solver is run a second time and generates an updated schedule of operations 8B for the automated laboratory equipment. As can be seen in **FIG. 3****,** in some examples, the updated schedule not only adds the operations of the newly received orders but also re-schedules operations of some of the orders that have been scheduled earlier but whose process has not been started yet. In this manner, the techniques of the present disclosure allow for scheduling both batch-wise order reception and continuously received orders.

During subsequent operation of the automated laboratory equipment, further orders might arrive. The schedule can be updated by re-running of the constraint optimization solver in response to one or more of these further orders. In one example, the schedule is continuously updated by re-running the constraint optimization solver in response to the receipt of each order. In other examples, the constraint optimization solver is only re-run after a predetermined number of orders (e.g., between 1 and 5000 or between 6 and 2000 orders) have been received.

In one example of batch processing, the constraint optimization solver runs a first optimization run after which a subset or none of the computed schedule or the complete schedule is fixed. The remaining subset of the schedule is released. Then all, a part of or none of the corresponding orders of the released part of the schedule are included in a next optimization run, whereby the fixed subset resulting from the first optimization run is taken into the next optimization run as resource non-availability.

A number of orders that are fixed or released again for the next batch as well as the batch-size can be constant or variable for each batch run. In some situations, e.g., due to a very early trigger, all of the orders can be newly scheduled (for instance in case of an error or when an extremely urgent order arrives before the scheduled processes have started).

Not all of the orders from the portion of the schedule that is not fixed need to be taken in for a new schedule. For instance, when a new set of high-priority orders might arrive and the solver might be re-run with a limited number of orders (e.g., ten orders) only. In this case, the new high-priority orders might be included first, and the set can be filled up with old orders.

In the example of **FIG. 3****,** the second run (or any further run) of the constraint optimization solver is triggered by a receipt of one or more new orders. In addition or alternatively, other events can trigger the re-running of the constraint optimization solver to generate an updated schedule.

In one example, the constraint optimization solver is re-run after a predetermined time has elapsed.

In other examples, a re-run can be triggered by detection of one or more external or internal conditions of the automated laboratory equipment. For instance, the operator or system may switch resources off or on, or resources can be exchanged during servicing, changing or exchanging the system. In these cases, the constraint optimization solver can be re-run to generate a new schedule with reduced, added or changed resources. In other examples, if an error occurs (e.g., a break-down of a particular resource of the automated laboratory equipment) this may automatically be signaled to the scheduler and a new schedule can be generated in response. In other examples, an internal condition can be that one or more components of the automated laboratory equipment do not work properly.

An external condition can include a workload or status of other automated laboratory equipment or the laboratory environment the automated laboratory equipment is located in (e.g., a number of lab personnel present at a predetermined time).

In response to these trigger conditions, the resource descriptions (or other parts of the model) and/or the objective functions can be changed. For instance, a resource description of automated laboratory equipment can be changed to account for a resource (e.g., a pipette head) which has broken down. Moreover, an objective function can be changed and an initial objective of "maximize" throughput may be changed for "save as many orders as possible". In some cases, e.g., if an error has occurred, not all orders may be successfully processed. In this case some orders may be sacrificed (terminated) to free resources to process other, possibly more important orders.

In still other examples, the constraint optimization solver is re-run after the objective function has been changed. This might happen upon user input or in response to a detection of a particular state of the automated laboratory equipment or the laboratory environment.

This list of examples shows that the techniques of the present disclosure can allow for a flexible adaptation of automated laboratory equipment to different situations. This might improve different aspects of the operation of the automated laboratory equipment (e.g., throughput, time to result, walk away time and resource consumption). In general, the triggering of re-runs can happen automatically (i.e., without involvement of an operator of the automated laboratory equipment).

In the previous sections, examples of a run-time execution of the techniques of the present disclosure have been discussed. In other examples, the generation of the schedule can take place during design- or configuration-time of the automated laboratory equipment.

In still other examples, the generation of the schedule of operations takes place in a simulation of the automated laboratory equipment. This case will be discussed subsequently.

As already explained above, the techniques of the present disclosure can include using models including resource descriptions of the automated laboratory equipment and protocols which can be independent of the (expected or desired) schedule of operations. In this manner, a user can modify the characteristics of the model (e.g., the resource descriptions and the protocol descriptions) and run the constraint optimization solver to simulate the behavior of automated laboratory equipment. Thus, new protocols for the automated equipment or laboratory workflows can be developed and tested for newly designed instruments as well as for existing instruments (e.g., after an instrument update with additional or changed protocols).

In addition or alternatively, new and/or modified automated laboratory equipment can be simulated. For instance, a user might add a component (e.g., an additional pipettor) to a model of existing automated laboratory equipment. Using the modified model as input for the constraint optimization solver, the behavior of the laboratory equipment can be studied.

In still other examples, characteristics of existing laboratory equipment can be studied by simulation. For instance, performance bottle-necks of existing laboratory equipment can be identified in schedules generated by the techniques of the present disclosure. In addition or alternatively, resource consumption (e.g., reagents or other consumable items) of an existing laboratory equipment can be estimated based on the schedules generated by the techniques of the present disclosure.

In the previous sections numerous use cases according to the present disclosure have been presented. Subsequently, aspects of the model will be discussed in connection with **FIG. 4** to **FIG. 7****.**

These sections also include code examples for the sake of illustration. The code is written in a form to be processed by the IBM ILOG CPLEX Optimizer. The IBM ILOG CPLEX Optimizer solves integer programming problems, large linear programming problems using either primal or dual variants of the simplex method or the barrier interior point method, convex and non-convex quadratic programming problems, and convex quadratically constrained problems (solved via second-order cone programming, or SOCP). However, the techniques of the present disclosure are not limited to this particular (generic) constraint optimization solver. Rather, the model and the objective function can also be implemented to be processed by other (generic) constraint optimization solvers (as listed above).

The protocol descriptions will be discussed first. **FIG. 4** depicts an example protocol description according to the present disclosure. The protocol described by the protocol description can relate to any assay or other test on a sample the automated laboratory equipment can carry out. In other examples, the protocol can relate to a preparation or manipulation of a sample. In still other examples, a protocol can relate to a cleaning, testing or maintenance operation of the automated laboratory equipment.

As can be seen in **FIG. 4****,** the protocol description includes a description of a plurality of protocol steps 31A-31D included in the protocol, and one or more constraints 32A-32G to be observed when carrying out the protocol steps on the automated laboratory equipment.

The protocol steps 31A-31D can include any operation the automated laboratory equipment can perform. These operations include transport of samples, reagents or consumable items (e.g., reaction vessels or instrument tips), manipulation of samples or reagents (e.g., sampling of a predetermined quantity of a reagent or sample), homogenization of fluid mixtures, incubating of fluids, measurement of a parameter of the sample, cleaning operations of reaction vessels or instrument components and disposal operations of non-renewable resources (e.g., fluids and consumables).

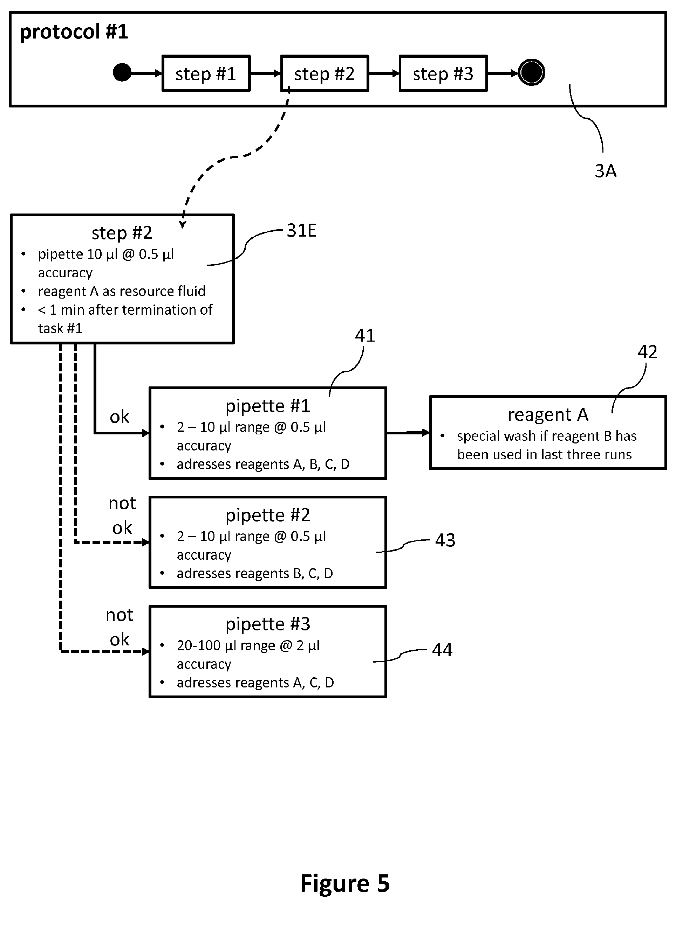

One example of a protocol step is given in **FIG. 5****.** Step #2 31E involves pipetting 10 µl of reagent A into a reaction vessel. In other examples, the description of the protocol steps can include other and/or further information (e.g., the duration of one or more protocol steps or an optionality flag indicating that a particular protocol step can be skipped).

A protocol step 31A-31D can combine multiple operations of the automated laboratory equipment. For example, step #1 might include adding a predetermined quantity of a sample and a reagent in a reaction vessel.

The constraints 32A-32G included in the protocol descriptions can include one or more of a definition of the sequence of protocol steps 32E-32G, a definition of the minimum and/or maximum duration of a protocol step 32C, 32D or a definition of the minimum and/or maximum time between two subsequent protocol steps 31A-31D. These subsequent protocol steps can be but not necessarily have to place directly after each other. For instance, in the example of **FIG. 5** step #2 must start at most one minute after step #1 has been completed.

A code example of a protocol description for a prothrombin time assay is given in the left hand column of **Table 1** below. The right hand column includes an explanation of various features of the protocol description. As can be seen, the protocol description includes a protocol identifier (i.e., "PT" in the example). Moreover, the protocol description defines a sequence of the steps and various constraints to be observed.

**Table 1**

| | |
|---|---|
| «"PT", "AspireS">, 18, 0>, | Protocol "PT" requires a step named "AspireS" with execution time 1.8s (all times are expressed as multiples of 100ms). This step is obligatory (optionality flag set to zero). The "SpecialWash" step is optional, marked by an optionality flag which is set to "1". |
| «"PT", "DummyDispS">, 9, 0>, | |
| «"PT", "WashNeedle">, 40, 0> | |
| <<"PT", "SpecialWash">, 100, 1>, | |
| <"PT", "AspireS", "DummyDispS", -1, 1200>, | Protocol "PT" does not require a minimum delay between the end of step "AspireS" and before starting the step "DummyDispS" (value "-1"). The maximum allowed waiting time between the steps is 120s. |
| <"PT", "Dispenses", | |
| "CuvPrepDispSR", 2562, 2582>, | The allowed time delay between the steps "DispenseS" and "CuvPrepDispSR" is at least 256.2s and at most 258.2s. |
| <"PT", "CuvLoad", "CuvToIncubator", -1, -1> | |
| | Step "CuvLoad" has to be scheduled before "CuvToIncubator" but there are no required times for the minimal nor maximum delay the two steps (indicated by "-1"). |

The resource descriptions will be discussed next. In general, the resource descriptions describe the resources present in the automated laboratory equipment and capabilities and constraints of these resources.

Resources of laboratory equipment can include, e.g., one or more of samplers and dispensers of samples and reagents (e.g., pipettors), grippers or other movers for manipulation of vessels, incubators, measurement units, cleaning stations and feeding devices for consumable items (e.g., reaction vessels, sample vessels or parts of the instrument).

An example for resource descriptions is shown in **FIG. 5****.** For the sake of illustration, four resource descriptions 41-44 are shown. On the one hand, the example laboratory equipment includes three pipettes. For each of these pipettes, the resource description 41-43 defines a range of volumes the respective pipettes can handle, an accuracy of the pipette, and a set of reagents the pipettes can address. In the same manner, a resource description of reagent A defines a constraint: A special wash step is required in case if another predefined reagent has been used a predetermined number of runs prior to the reagent A.

In addition, in some examples, the resource descriptions can define one or more of states in which the resource can be, processes that cause a transition from one state (level) to the next, eventually a transition time required to transition between states of the resource, requirements of states of the resource to carry out a predetermined protocol step, and incompatibilities between different states of the resource.

In one example, the resource description includes different components. For instance, the resource description can include a resource definition component which defines the capacity of the respective resource, a component describing how the resource can be occupied and released by predetermined protocol steps, a resource state component which defines a dynamic behavior of the respective resource and a component defining rules to be obeyed in certain situations.

In one example, the resource definition component defines a resource identifier and a capacity of the resource. For instance, the number of resources of a certain type available in the instrument can be defined. Resources may be separately defined (e.g., the pipettes in **FIG. 5****)** as having a capacity of one, or as a joint set of resources in one unit (e.g., an incubator with a capacity of multiple cuvette positions). As shown in **FIG. 5****,** the resource definition component can specify additional characteristics of the respective resource. A code example for a resource definition component is shown in **Table** 2 below:

**Table 2**

| | |
|---|---|
| <"NeedleSample", 1>, | The automated laboratory equipment includes one needle unit for the samples ("NeedleSample") and a second needle unit for reagents ("NeedleReagent"). Furthermore, there are twelve positions for cuvettes in a photometer unit and twenty positions in an incubator. |
| <"NeedleReagent", 1>, | |
| <"PhotometerPos", 12>, | |
| <"IncubatorPos", 20>, | |

The component describing how the resource can be occupied and released by predetermined protocol steps can define one or more of respective resources required for a particular protocol step, actions necessary to release a resource after it has been used in a particular protocol step and a constraint regarding a maximum number of resources that can be used. A code example for this component is shown in **Table 3** below:

**Table 3**

| | |
|---|---|
| <"NeedleSample", "AspireS", 1>, | Protocol step "AspireS" occupies one sample needle. There might be multiple needles of the type "Sample". |
| <"PhotometerPos", "CuvMoveIncub", 1>, | One photometer position is used to place a cuvette in the photometer. |
| <"NeedleSample", "WashNeedle", 1>, | Protocol step "WashNeedle" releases one sample needle. Until a wash has been carried out the needle cannot be used because it is contaminated. |
| <"PhotometerPos", "CuvMoveTrash", 1>, | Protocol step "CuvMoveTrash" releases one photometer position as the cuvette is taken out of the photometer and disposed. |
| photometerResourceUse[resCurr] <= resCurr.nCapacity; | Definition of the maximum allowed resource use: the current resource use shall be less or equal to the capacity. |

The resource state component can define one or more of states the respective resource can be in, a required transition time between different states, a required state for a particular protocol step and information regarding incompatibility between different states. A code example for this component is shown in **Table 4** below:

**Table 4**

| | |
|---|---|
| <"TransferArm", "ReagentCassette">, <"TransferArm", "Incubator">, | The resource "TransferArm" has two possible spatial positions: the first position being above reagent cassettes, the second position being above the incubators. |
| <"TransferArm", "ReagentCassette", "Incubator", 11>, | The resource "TransferArm" needs 1.1s for moving from the position "ReagentCassette" to the position "Incubator". |
| <"AspireReagent", "TransferArm", "ReagentCassette">, | In order to carry out the protocol step "AspireReagent", the resource "TransferArm" needs to be in the position "ReagentCassette". |
| <<"TransferArm_1", "ReagentCassette_a1">, <"TransferArm_2", "ReagentCassette_a2"» | The resource "TransferArm_1" and cannot be in state "ReagentCassette_a1" if, at the same time, resource "TransferArm_2" is in state "ReagentCassette_a2". The reverse direction is also considered. |

Last, the component defining rules to be obeyed in certain situations can define different exceptions to be applied in particular situations. For instance, this component can define rules to avoid carry over (e.g., contamination of a sample or a reagent with another sample, reagent or assay) during the operation of the automated laboratory equipment.

In one example, carry-over can be avoided by implementing one or more of: Adding one or more special cleaning steps to the schedule (e.g. with special cleaning agents) between two steps between which carry-over might occur, changing a processing order of two or more tasks between which carry-over might occur (sensitive task before contaminating task) and carrying out a predetermined number of steps (e.g. normal rinsing rather than special cleaning steps) between two tasks that cause carry-over.

A code example for this component is shown in **Table 5** below:

**Table 5**

| | |
|---|---|
| <"CarryOver_R1", 100>, | There is a carry-over type called "CarryOver_R1". Its maximum (ceiling) level is 100 units (the contamination of the resource is measured in these units). |
| <<"CAUSE", "AspireR1_CarryOver">, <"CarryOver_R1" , 10», | Protocol step "AspireR1_CarryOver" from protocol "CAUSE" increases the level of "CarryOver R1" by ten units (until ceiling value of 100 is reached). |
| <<"Sensitive", "SpecialWash_R1">, <"CarryOver_R1" , 0>>, | Protocol step "SpecialWash_R1" from protocol "Sensitive" resets the level of "CarryOver R1" to zero. |
| <<"PT", "WashNeedle">, <"CarryOver_R1" , -1», | Protocol step "WashNeedle" from protocol "PT" reduces the level of "CarryOver_R1" by at most one unit. |
| <<"Sensitive", "AspireR1">, <"CarryOver_R1" , 0>>, | Protocol step "AspireR1" from protocol "Sensitive" requires the level of "CarryOver_R1" to be at most zero in order to be executed. |

After having discussed different aspects of the resource descriptions of the model, the following sections will treat the order list and the objective function in more detail.

The order list includes information regarding at least one order (e.g., a plurality of orders) to be carried out by the automated laboratory equipment, each order involving the execution of one or more protocols. For instance, each order can specify an assay to be performed on one sample or a batch of samples. In other examples, each order can specify a plurality of assays to be performed on one sample or a batch of samples.

In one example, the order list includes for each order one or more of a sample identifier to identify a sample or a batch of samples (e.g., a barcode attached to a sample container), a protocol identifier identifying one or more protocols to be applied to the sample or batch of samples and an entry time indicating a time at which the order entered the system.

In addition or alternatively, the order list can include for each order an expiration time indicating a time or duration until which the sample or batch of samples must be processed. Alternatively or in addition, the order list can include for each order a priority indicator indicating a priority value of a sample or batch of samples. The latter two pieces of information can form constraints for the solver and can be used to prioritize certain samples or batches of samples. A code example for an order list including three orders is shown in **Table 6** below:

**Table 6**

| | |
|---|---|
| <<1, "PT" >, 0, 72000>, | First, at time = 0 s two orders for sample with ID = 1 entered on which a PT (prothrombin time) and APTT (activated partial thromboplastin time) test shall be carried out. Both tests shall be finished latest 72000 * 100 ms = 2 hours after entry time of the sample. Next a sample arrived at time = 35s with ID = 2, for which a PT test needs to be carried out finishing not earlier than 30 minutes after the entry time. |
| <<1, "APTT" >, 0, 72000>, | |
| <<2, "PT" >, 350, 18000> | |

In the subsequent sections the objective function will be discussed in more detail. In general, the objective function defines an optimization goal for a schedule of operations for the automated laboratory equipment.

An objective function defines an objective and the quality of a generated schedule can be judged according to the degree it reaches the objective. The objective function quantifies the objective so that in some cases minima or maxima of the objective function have to be found by the constraint optimization solver. A certain schedule might not be an absolute maximum or minimum of the objective function but nevertheless be a useful result. In addition, the constraint optimization solver can perform multiple runs to improve the found solution.

The objective function can be adjusted to fulfill different optimization goals. The optimization goals can include minimizing one or more of a total time or duration to process one or a plurality of orders (e.g., of a batch of orders or multiple batches of orders), maximizing a throughput of automated laboratory equipment, minimizing a resource consumption (e.g., reagents, cleaning agents, or other consumable items, energy or renewable resources (instrument resources)) of the automated laboratory equipment, distributing the workload evenly over different components of the automated laboratory equipment or different automated laboratory equipment and minimizing a number of (high-priority) orders lost in case of an error occurring in the automated laboratory equipment.

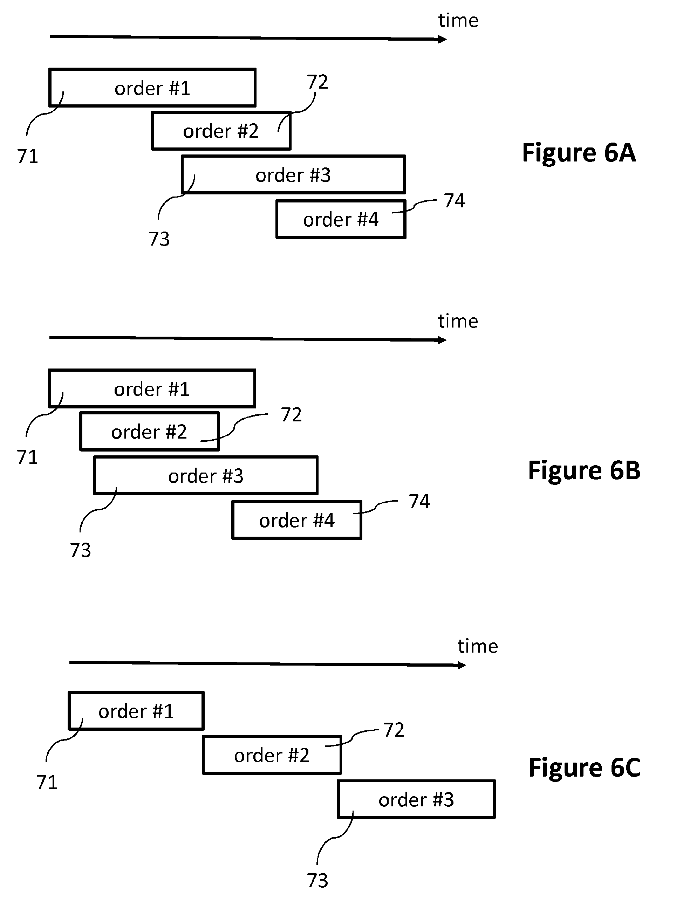

In this manner, the schedule can be flexibly adapted to different optimization goals (as also discussed above). Examples of schedules generated by using different objective functions are shown in **FIG. 6A** to **FIG. 6C****.** In all examples, a batch of three or for orders 71-74 shall be scheduled. The total durations of the processes per order are shown. In the first example of **FIG. 6A** might provide the best long term throughput even though the throughput of the schedule of **FIG. 6B** is higher in the short run. The schedule of **FIG. 6C** might yield to lowest processing time for each order, even though the short term throughput is lower.

In one example, the objective function includes a weighted sum of two or more factors, wherein each factor quantifies an optimization goal. For instance, a weighted function could include a first term quantifying a total throughput of the automated laboratory equipment and a second term quantifying a resource consumption. In this example, the first term could be weighted with a larger weight factor to ensure that throughput is optimized in the first place. The second term could be weighted with a lower, negative weight factor to ensure that very high resource consumption is avoided. The overall objective function can be maximized by the constraint optimization solver to generate the schedule.

A code example for an objective function is shown in **Table 7** below:

**Table 7**

| | |
|---|---|
| minimize | One part of the objective function contain a term expressing the weighted penalty for the total execution time (called make span), as well as a fairness penalty term considering the total execution time of the single orders. The direction of optimization is a minimization. |
| weightedMakeSpan + weightedFairnessPenalty | |

As part of the process of generating the schedule, the resource descriptions and protocols descriptions of the model can be linked. This might happen automatically or by user intervention. As described in the preceding sections, different parts of the model and the order list can include identifiers of different steps the automated laboratory equipment can perform (e.g., a "WashNeedle" step occurring above). In some examples, these identifiers can be used to link the respective parts of the model as part of the schedule generation process.

**FIG. 5** exemplifies such linking process. A protocol description 3A can define three steps. One of these steps (step #2) 31E involves pipetting a quantity of a reagent with a predefined accuracy. Now, it can be found in the resource description of the model that only one of the three pipettes 41 is capable of performing the required pipetting step and there is only one instance of the necessary reagent. Therefore, the step of the protocol is linked to the resources automatically in the course of the schedule generation or by a user. In other examples more than one resource may be applicable to carry out the protocol step. In this case, the solver will find which of the group of applicable resources is most suitable to maximize or minimize the objective function.

In the preceding sections, the elements which are used in the schedule generation process according to the present invention have been discussed. In the following sections, in connection with **FIG. 7****,** an example automated laboratory device for which a schedule can be generated will be discussed.

As can be seen in **FIG. 7****,** the example automated laboratory equipment is an automated analyzer 101. The automated analyzer includes a reagent repository 102 including a plurality of reagents 104 (e.g., packed in standard test-specific cassettes with a plurality of reservoirs) and a sample repository 103 including a plurality of samples 105 (in, e.g., sample collection tubes). The automated analyzer 101 further includes a set of multiple pipettes 106 (e.g., three pipettes) which can be moved in all three spatial directions. Moreover, the automated analyzer 101 includes multiple incubator positions 109 and multiple measurement channels 110. The incubation and measurement operations take place in disposable reaction vessels (called "cuvettes") which are supplied through a cuvette supply 111 and disposed in a cuvette waste 113. The manipulation of the cuvettes can be effected by two grippers 112, 114, one of the grippers 112 having also a mixer function via shaking the cuvette. Last, the automated analyzer 101 includes one or two wash stations 107, 108 configured to wash the pipettes 106. A batch of sample tubes with related orders arriving at the automated analyzer can each include providing a cuvette at an measurement position 110 for measuring the cuvette quality and/or blank-measurement (a first step of the protocols), pipetting a quantity of a sample 105 into the cuvette (a second step of the protocols) and adding a predetermined reagent 104 (a third step of the protocols) after which the mixture is homogenized by the mixer-gripper Then, the cuvette is placed in the incubator 109 to incubate the sample/reagent mixture for a predetermined period of time (a fourth step of the protocols). Next, the cuvettes are transferred to pipette start reagent and to place it into a measurement position 110 (a fifth step of the protocols) where a measurement takes place (a sixth and seventh steps of the protocols). Last, the cuvette is disposed (a final step of the protocols). As can be seen, already the relatively simple protocol described herein requires scheduling the operations of a plurality of resources of the automated analyzer. This can be done by using the techniques according to the present disclosure.

Even though a particular automated analyzer is shown in **FIG. 7****,** the techniques of the present disclosure are not limited in any way to this particular analyzer. Rather, the aspects of the elements discussed above (e.g., the model, the order list and the objective function as well as the application examples) can also be used for other automated laboratory equipment (e.g., the automated laboratory equipment discussed in the summary section).

In addition, the techniques of the present disclosure are not limited to a single automated laboratory device. Rather, a schedule for a plurality automated devices can also be generated in a similar way as described herein for a single automated laboratory device. For example, in this case the model can include resource descriptions for the resources of each of the plurality of automated laboratory devices.

In still other examples, a schedule for a laboratory environment including automated laboratory equipment can also be generated in a similar way as described herein for a single automated laboratory device. For example, in this case the model can include resource descriptions for the resources of the laboratory environment and protocol descriptions describing workflows in the laboratory environment.

In the following sections, several aspects of the hardware generating the schedules according to the present invention will be discussed. In general, the operations for generating the schedule can be carried out on each suitable computer system (e.g., including a single or multiple computers) or on a plurality of computer systems linked by a communication network.

In one example, the computer system can be integrated in the automated laboratory equipment (e.g., as part of a controller of an automated laboratory device). In this example, the schedule can be generated locally at the automated laboratory equipment.

In other examples, the computer system can be arranged remotely from the automated laboratory equipment for which a schedule is generated. For example, the computer system can be part of a laboratory environment and networked to the automated analyzer through a laboratory network.

In still other examples, the computer system can be remote from the laboratory environment. For instance, the constraint optimization solver can be running on a computer system which is connected to the automated laboratory equipment or the laboratory environment including the automated laboratory equipment for which a schedule is generated through the internet.

In addition or alternatively, the constraint optimization solver can be running on a distributed computer system (e.g., a cloud-based computer system).

If the generation of a schedule for automated laboratory equipment is simulated, there is no need for the automated laboratory equipment to be actually networked with the computer system doing the simulation. In fact, as discussed above, the techniques of the present disclosure can be used in the design phase of automated laboratory equipment so this equipment might not even exist.

In the preceding detailed description multiple examples of methods and systems for providing a schedule of operations for automated laboratory equipment of the present disclosure have been discussed. However, the methods and systems for providing a schedule of operations for automated laboratory equipment of the present disclosure can also be configured as set out in the following aspects:
1. A computer-implemented method for providing a schedule of operations for automated laboratory equipment, the method comprising:
   receiving at least one order,
   wherein each order requires the execution of one or more protocols on the automated laboratory equipment;
   generating one or more optimization problem instances using one or more resource descriptions of the automated laboratory equipment, one or more protocol descriptions of the automated laboratory equipment, an objective function and the at least one order,
   wherein the one or more resource descriptions describe the resources present in the automated laboratory equipment and capabilities and constraints of the resources;
   wherein each protocol description describes a protocol the automated laboratory equipment can carry out,
   wherein each protocol description includes a definition of one or more protocol steps to be carried out by the automated laboratory equipment, and
   wherein each protocol description includes one or more constraints to be observed when carrying out the protocol steps on the laboratory equipment,
   wherein the objective function defines an optimization goal for a schedule of operations for the automated laboratory equipment;
   the method further comprising:
   providing the one or more optimization problem instances as input to a constraint optimization solver; and
   processing the one or more optimization problem instances by the constraint optimization solver to generate the schedule of operations to carry out the at least one order on the automated laboratory equipment.
2. The method of aspect 1, wherein the processing of the one or more optimization problem instances by the constraint optimization solver takes place during run-time of the automated laboratory equipment.
3. The method of aspect 2, wherein the processing of the one or more optimization problem instances by the constraint optimization solver takes place during run-time of the at automated laboratory equipment in response to a triggering event.
4. The method of aspect 3, wherein the triggering event is one of:
   an arrival of one or more new orders, a change in the resources of the automated laboratory equipment, a user input, a modification of an order forecast, a lapse of a predetermined period of time, an error, an exceptional situation, or a combination of two or more of these events.
5. The method of aspect 4, wherein the triggering event is the arrival of an order having a high priority.
6. The method of aspect 4, wherein the user input is a user-defined change of operation, e.g., a turning off certain resources, a user-defined prioritization of orders, a user-defined assignment or interdiction of using certain resources for certain protocols or orders, an application for user-defined protocols or protocol or resource parameter changes by a user.
7. The method of any one of aspects 3 to 6, wherein the processing of the one or more optimization problem instances by the constraint optimization solver is repeated automatically in response to the occurrence of a respective triggering event.
8. The method of any one of the preceding aspects, further comprising re-running the constraint optimization solver during run-time of the automated laboratory equipment to generate an updated schedule of operations.
9. The method of any one of the preceding aspects, further comprising:
   collecting a batch of multiple orders for the automated laboratory equipment; and
   processing the optimization problem instance by the constraint optimization solver to generate a schedule of operations for the batch of multiple orders at the same time.
10. The method of aspect 9, wherein a number of orders in the batch is between 1 and 5000, optionally between 6 and 2000.
11. The method of aspect 1, wherein the processing of the one or more optimization problem instances by the constraint optimization solver takes place during development- or design-time of instrument software of the automated laboratory equipment.
12. The method of aspect 1, wherein the processing of the one or more optimization problem instances by the constraint optimization solver takes place during development- or design-time of schedules of operations of the automated laboratory equipment.
13. The method of any of the preceding aspects 11 or 12, further comprising applying the schedule generated during development or design time on the automated laboratory equipment as a pre-defined schedule to be used during run-time of the automated laboratory equipment.
14. The method of any of the preceding aspects, wherein method is carried out to simulate a behavior of the automated laboratory equipment, wherein the at least on order is at least one hypothetical order for the automated laboratory equipment and the schedule of operations is a schedule for the at least one hypothetical order.
15. The method of any of the preceding aspects, wherein method is carried out to predict one or more of a required time for at least one order, an expected time of arrival of results and a lab performance including error situations.
16. The method of any of the preceding aspects, further comprising changing the one or more resource descriptions, the one or more protocol descriptions and/or the objective function automatically by the automated laboratory equipment during operation.
17. The method of aspect 16, wherein the automatic change happens in response to detecting that resources are not available, an error modus or state change of the automated laboratory equipment.
18. The method of any one of the preceding aspects, wherein continuously arriving orders are scheduled by sequential batch-wise optimizations.
19. The method of aspect 18, comprising:
   running a first optimization run, wherein a subset or none of the computed schedule or the complete schedule is fixed and the remaining subset of the schedule is released and all, a part of or none of the corresponding orders of the released part of the schedule are included in a next optimization run, whereby the fixed subset resulting from the first optimization run is taken into the next optimization run as resource non-availability.
20. The method of aspect 19, wherein a number of orders that are fixed or released again for the next batch as well as the batch-size may be constant or vary for each batch run.
21. The method of any one of the preceding aspects, further comprising:
   providing the schedule of operations to a controller of the automated laboratory equipment; and
   instructing, by the controller, the automated laboratory equipment to carry out the operations according to the schedule.
22. The method of aspect 21, wherein the providing and the instructing steps take place automatically.
23. The method of any one of the preceding aspects, further comprising providing the schedule for display on a graphical user interface.
24. The method of any one of the preceding aspects, wherein the optimization goal is one of the list of optimizing a throughput of the automated laboratory equipment, processing one or more particular order as fast possible, using as little of a predetermined resource as possible, balancing of workload of two or more resources, or a combination of these goals.
25. The method of aspect 24, wherein the objective function includes a weighted or prioritized combination of different optimization goals.
26. The method of any one of the preceding aspects, wherein the automated laboratory equipment includes a plurality of automated laboratory devices,
   wherein the orders are orders to be carried out by a plurality of automated laboratory devices,
   wherein the constraint optimization solver processes the one or more optimization problem instances to generate a schedule operations for the plurality of automated laboratory devices.
27. The method of aspect 26, wherein the plurality of automated laboratory devices include two or more automated laboratory devices of the same type.
28. The method of any one of the preceding aspects, wherein the automated laboratory equipment is part of a laboratory environment,
   wherein the orders are orders to be carried out in the laboratory environment,
   wherein the generic constraint optimization solver processes the one or more optimization problem instances generate a schedule of workflows in the laboratory environment.
29. The method of any one of the preceding aspects, wherein the automated laboratory equipment includes an automated analyzer for samples.
30. The method of aspect 29, wherein the automated analyzer for samples is an in-vitro analyzer.
31. The method of aspect 30, wherein the in-vitro analyzer is one of a clinical chemistry analyzer, an immune-chemistry analyzer, an analyzer for ion selective analysis, a hematology analyzer, a coagulation analyzer, a nucleic acid analyzer, a sequencer, a blood gas analyzer , a mass spectrometer, a cytometer, or a combination of two or more of these analyzers.
32. The method of any one of aspects 1 to 31, wherein the automated laboratory equipment includes one or more of a transportation system configured to transport non-renewable resources such as samples, reagents, reaction vessels, multi-well plates, pipetting tips, slides, consumables, waste or renewable resources such as sensor or analysis modules or modules that can process non-renewable resources in a laboratory environment.
33. The method of any one of aspects 1 to 32, wherein the automated laboratory equipment includes one or more pre-analysis systems, e.g., aliquoting systems, tube capping and de-capping systems, tube or well-plate sealers and de-sealers, sample sorting systems or sample re-formatting systems.
34. The method of any one of aspects 1 to 33, wherein the automated laboratory equipment includes one or more sample post-analysis systems, e.g., sample / specimen archiving systems, systems for preparing samples / specimens for storage, waste handling systems, or cleaning systems.
35. The method of any of the preceding aspects, wherein generating one or more optimization problem instances includes using a model of automated laboratory equipment which is specified by the one or more resource descriptions of the automated laboratory equipment and the one or more protocol descriptions of the automated laboratory equipment.
36. The method of any one of the preceding aspects, wherein the resource descriptions define which protocol steps can be carried out by a particular resource or a group of resources of the automated laboratory equipment.
37. The method of any one of the preceding aspects,
   wherein the resource descriptions include descriptions groups of resources with specific characteristics; and
   wherein the protocol descriptions define one of more characteristics of the resources which can be requested by a protocol step.
38. The method of any one of the preceding aspects, wherein the resource descriptions define one or more of:
   states in which the resource can be, a transition time required to transition between states of the resource, requirements of states of the resource to carry out a predetermined protocol step, and incompatibilities between different states of the resource.
39. The method of any one of the preceding aspects, wherein the orders define one or more of:
   a sample ID of a particular sample, a protocol identifier identifying an assay to be performed on a sample, an entry time of the order and an expiration time of the order.
40. The method of any one of the preceding aspects, wherein the protocol descriptions include one or more of:
   protocol identifiers, durations of the tasks in the protocol, sequences of the tasks in the protocol, timing windows for a durations of tasks and timing windows for times between two tasks.
41. The method of any one of the preceding aspects, wherein generating one or more optimization problem further comprises using a definition of one or more exceptions which define how the automated laboratory equipment should handle one or more particular situations.
42. The method of aspect 41, wherein the one or more particular situations include a situation in which a substance associated with a first order is carried over to a process of another order.
43. The method of aspect 41, wherein the one or more particular situations include a situation in which a resource is in state after having carried out a particular which could affect the processing of other subsequent tasks.
44. The method of any one of aspects 41 to 43, wherein the one or more exceptions define one or more of:
   that one or more cleaning or other resource-conditioning steps should be added to the schedule, that a processing order of two or more tasks shall be changed and that a predetermined number of tasks shall be carried out between two tasks which are sensitive to carry-over.
45. The method of any one of the preceding aspects, wherein the method includes leaving gaps in the schedule which can be filled later upon arrival of new orders.
46. The method of any one of the preceding aspects, generating one or more optimization problem instances includes using a forecast of expected orders.
47. The method of any one of the preceding aspects, wherein the constraint optimization solver is a generic constraint optimization solver.
48. The method of any one of the preceding aspects, wherein the protocol descriptions are defined independently of potential solutions for the schedule of operations.
49. The method of any one of the preceding aspects, wherein the resource descriptions are defined independently of potential solutions for the schedule of operations.
50. The method of any one of the preceding aspects, wherein as a starting schedule solution for the constraint optimization solver either pre-defined pattern, optionally an optimal schedule for routine tests defined at design time, or patterns of schedule solutions that have been previously calculated is used.
51. The method of any one of the preceding aspects, further comprising:
   defining one or more constraints to force the constraint optimization solver to search for solutions with repetitive patterns,
   wherein processing the one or more optimization problem instances include forcing the schedule to fit a repetitive pattern.
52. The method of claim 51, further comprising uploading the repetitive pattern to the automated analyzer and using the repetitive pattern at run-time to automatically fit the ordered protocols on the repetitive pattern.
53. A laboratory network, the laboratory network including:
   automated laboratory equipment; and
   a computer system configured to carry out the steps of any one of the methods of aspects 1 to 52 to generate the schedule of operations for the automated laboratory equipment.
54. The laboratory network of aspect 53, wherein the computer system is a part of the automated laboratory equipment.
55. The laboratory network of aspect 53, wherein the computer system is remote from the automated laboratory equipment.
56. The laboratory network of any one of aspects 53 to 55, wherein the steps of any one of the methods of aspects 1 to 50 are at least partially carried out on a remote computer device or by a cloud-based application.
57. The laboratory network of any one of aspects 53 to 56, wherein the laboratory network includes a plurality automated laboratory instruments and wherein the schedule of operations includes orders for the plurality of automated laboratory instruments.
58. The laboratory network of any one of aspects 53 to 57, wherein the automated laboratory instrument includes an automated analyzer for samples.
59. The method of aspect 58, wherein the automated analyzer for samples is an in-vitro analyzer.
60. The method of aspect 59, wherein the in-vitro analyzer is one of a clinical chemistry analyzer, an immune-chemistry analyzer, an analyzer for ion selective analysis, a hematology analyzer, a coagulation analyzer, a nucleic acid analyzer, a sequencer, a blood gas analyzer , a mass spectrometer, a cytometer, or a combination of two or more of these analyzers.
61. The laboratory network of any one of aspects 53 to 60, wherein the automated laboratory instrument includes a controller configured to carry out the operations according to the schedule.
62. A computer-readable medium including instructions thereon, which when executed by a computer system make the computer system carrying out the steps according to any one of methods 1 to 52.

Further disclosed and proposed is a computer program including computer-executable instructions for performing the method according to the present invention in one or more of the embodiments enclosed herein when the program is executed on a computer or computer network. Specifically, the computer program may be stored on a computer-readable data carrier. Thus, specifically, one, more than one or even all of method steps as disclosed herein may be performed by using a computer or a computer network, preferably by using a computer program.

Further disclosed and proposed is a computer program product having program code means, in order to perform the method according to the present invention in one or more of the embodiments enclosed herein when the program is executed on a computer or computer network. Specifically, the program code means may be stored on a computer-readable data carrier.

Further disclosed and proposed is a data carrier having a data structure stored thereon, which, after loading into a computer or computer network, such as into a working memory or main memory of the computer or computer network, may execute the method according to one or more of the embodiments disclosed herein.

Further disclosed and proposed is a computer program product with program code means stored on a machine-readable carrier, in order to perform the method according to one or more of the embodiments disclosed herein, when the program is executed on a computer or computer network. As used herein, a computer program product refers to the program as a tradable product. The product may generally exist in an arbitrary format, such as in a paper format, or on a computer-readable data carrier. Specifically, the computer program product may be distributed over a data network.

Further disclosed and proposed is a modulated data signal which contains instructions readable by a computer system or computer network, for performing the method according to one or more of the embodiments disclosed herein.

Referring to the computer-implemented aspects of the invention, one or more of the method steps or even all of the method steps of the method according to one or more of the embodiments disclosed herein may be performed by using a computer or computer network. Thus, generally, any of the method steps including provision and/or manipulation of data may be performed by using a computer or computer network. Generally, these method steps may include any of the method steps, typically except for method steps requiring manual work, such as providing the samples and/or certain aspects of performing measurements.

Further disclosed and proposed is a computer or computer network comprising at least one processor, wherein the processor is adapted to perform the method according to one of the embodiments described in this description.

Further disclosed and proposed is a computer loadable data structure that is adapted to perform the method according to one of the embodiments described in this description while the data structure is being executed on a computer.

Further disclosed and proposed is a storage medium, wherein a data structure is stored on the storage medium and wherein the data structure is adapted to perform the method according to one of the embodiments described in this description after having been loaded into a main and/or working storage of a computer or of a computer network.

## Claims

1. A computer-implemented method for providing a schedule of operations for automated laboratory equipment, the method comprising:
receiving at least one order (7),
wherein each order (7) requires the execution of one or more protocols on the automated laboratory equipment;
generating one or more optimization problem instances using one or more resource descriptions (4; 41-44) of the automated laboratory equipment, one or more protocol descriptions (3; 3A) of the automated laboratory equipment, an objective function (6) and the at least one order (7),
wherein the one or more resource descriptions (4; 41-44) describe the resources present in the automated laboratory equipment and capabilities and constraints of the resources;
wherein each protocol description (3; 3A) describes a protocol the automated laboratory equipment can carry out,
wherein each protocol description includes a definition of one or more protocol steps (31A-31E) to be carried out by the automated laboratory equipment, and wherein each protocol description includes one or more constraints (32A-32G) to be observed when carrying out the protocol steps on the laboratory equipment,
wherein the objective function (6) defines an optimization goal for a schedule of operations for the automated laboratory equipment;
the method further comprising:
providing the one or more optimization problem instances as input to a constraint optimization solver; and
processing the one or more optimization problem instances by the constraint optimization solver to generate the schedule of operations (8) to carry out the at least one order on the automated laboratory equipment.

2. The method of claim 1, wherein the processing of the one or more optimization problem instances by the constraint optimization solver takes place during run-time of the automated laboratory equipment.

3. The method of claim 2, wherein the processing of the one or more optimization problem instances by the constraint optimization solver takes place during run-time of the automated laboratory equipment in response to a triggering event, optionally wherein the triggering event is one of an arrival of one or more new orders, a change in the resources of the automated laboratory equipment, a user input, a modification of an order forecast, a lapse of a predetermined period of time, an error, an exceptional situation, or a combination of two or more of these events.

4. The method of any one of claims 1 to 3, wherein the processing of the one or more optimization problem instances by the constraint optimization solver is repeated automatically in response to the occurrence of a respective triggering event.

5. The method of any one of the preceding claims, further comprising:
collecting a batch of multiple orders (7) for the automated laboratory equipment; and
processing the optimization problem instance by the constraint optimization solver (2) to generate a schedule of operations (8) for the batch of multiple orders at the same time.

6. The method of claim 1, wherein the processing step takes place during development- or design-time of instrument software of the automated laboratory equipment, or wherein the processing step takes place during development- or design-time of schedules of operations of the automated laboratory equipment.

7. The method of any of the preceding claims, wherein the method is carried out to simulate a behavior of the automated laboratory equipment, wherein the at least one order (7) is at least one hypothetical order for the automated laboratory equipment and the schedule of operations is a schedule for the at least one hypothetical order.

8. The method of any of the preceding claims, further comprising changing the one or more resource descriptions (4; 41-44), the one or more protocol descriptions (3; 3A) and/or the objective function (6) automatically by the automated laboratory equipment during operation, optionally wherein the automatic change happens in response to detecting that resources are not available, an error modus, a user input or state change of the automated laboratory equipment.

9. The method of any one of the preceding claims, further comprising:
providing the schedule of operations (8) to a controller of the automated laboratory equipment; and
instructing, by the controller, the automated laboratory equipment to carry out the operations according to the schedule.

10. The method of any one of the preceding claims, wherein the optimization goal is one of the list of optimizing a throughput of the automated laboratory equipment, processing one or more particular orders as fast possible, using as little of a predetermined resource as possible, balancing of workload of two or more resources, or a combination of these goals.

11. The method of any one of the preceding claims, wherein the automated laboratory equipment includes a plurality of automated laboratory devices,
wherein the orders (7) are orders to be carried out by a plurality of automated laboratory devices,
wherein the constraint optimization solver (2) processes the one or more optimization problem instances to generate a schedule of operations for the plurality of automated laboratory devices.

12. The method of any one of the preceding claims, wherein the automated laboratory equipment is part of a laboratory environment,
wherein the orders (7) are orders to be carried out in the laboratory environment,
wherein the generic constraint optimization solver (2) processes the one or more optimization problem instances to generate a schedule of workflows in the laboratory environment.

13. The method of any one of the preceding claims, wherein the protocol descriptions (3;
3A) are defined independently of potential solutions for the schedule of operations and wherein the resource descriptions (4; 41-44) are defined independently of potential solutions for the schedule of operations.

14. A laboratory network, the laboratory network including:
automated laboratory equipment (101); and
a computer system configured to carry out the steps of any one of the methods of claims 1 to 13 to generate the schedule of operations for the automated laboratory equipment (101).

15. A computer-readable medium including instructions thereon, which when executed by a computer system make the computer system carrying out the steps according to any one of the methods of claims 1 to 13.
